# EUROPEAN PATENT SPECIFICATION

(11) **EP 2 098 165 B1**
(45) Date of publication and mention of the grant of the patent: **02.05.2018**
(21) Application number: 08152203.9
(22) Date of filing: 03.03.2008
(51) Int. Cl.: A61B 5/00, A61B 5/024, A61B 5/22, G06F 1/16, A61B 5/044

(54) **Multifunctional portable electronic device and method for transmitting a particular graphical user interface to the display of a multifunctional portable electronic device**
Multifunktionale tragbare elektronische Vorrichtung und Sendung einer bestimmten graphischen Benutzeroberfläche an die Anzeige einer multifunktionalen tragbaren elektronischen Vorrichtung
Dispositif électronique portable multifonction et procédé pour la transmission d'une interface graphique particulière pour afficher un dispositif électronique multifonction portable

(43) Date of publication of application: 09.09.2009
(73) Proprietor: Vodafone GmbH, 40549 Düsseldorf (DE)
(72) Inventor: Weisscher, Alard, 6231 LG Meerssen (NL)
(74) Representative: Cullinane, Marietta Bettina

(56) References cited:
- WO-A-01/28416
- US-A1- 2002 080 031
- US-A1- 2002 130 181
- US-B1- 7 206 602

## Description

The present invention relates to a multifunctional portable electronic device. Furthermore the present invention relates to a method for transmitting a particular graphical user interface to the display of a multifunctional portable electronic device.

At present when people are exercising outside, for example running or cycling, they often have several information and communication needs, like knowing the time, hearing music, having a emergency call opportunity, knowing the running or cycling speed or distance, knowing their heart rate, etc.. These several information and communication needs are at present addressed by different devices, for example a watch, a heart rate monitor, a mobile telephone or a mp3-player. This results in having to carry several devices that combined are considered heavy, constrain movements due to different methods of attachment, and are often poorly integrated, for example music does not switch of when the mobile telephone rings.

It is known that a portable electronic device, like a mobile telephone, comprises sensor means or the portable electronic device builds the hub for sensor means. Portable electronic devices comprise displays to present known and/or measured data and parameters. A mobile telephone for example can be used as bike tachometer for presenting the speed or the distance of the bike during a trip with the bike. Therefore the graphical user interface of the mobile telephone can be adapted to the needs of a bike tachometer. The user has to adjust the graphical bike user interface out of a menu.

Pulse monitors can be worn at the wrist of a person by using a wristband. The same pulse monitor can be fixed at a handle bar holder of a bike. Therefore the wristband has to be fixed at the handle bar holder. Pulse monitors can have the function of a tachometer, as well. On the display of such a pulse monitor the heart rate is shown in one part of the display and the speed or the distance is shown in another part of the display. In the prior art monitoring devices for athletes are known which measure the heart rate of an exerciser during a sports performance. Such a monitoring device is for example described in EP°1°195°135°B1. The monitoring device comprises an electrode belt placed on the chest and a wrist-wore device for processing the received signal and for display of the heart rate. An athlete can thereby monitor his heart rate on the display during exercise.

Further, mobile telephones are known, whereby the display is detachable. The EP°1°318 650°B1 disclose a foldable mobile telephone with a detachable display. It is known that the detachable display stays in contact with the main unit after being detached. The contact can be wireless.

The WO 01/28416 A1 discloses a computer comprising at least one separate sensor for measuring physical parameters, transmission means for receiving the measured physical parameters, a battery, and at least one separate fixating device. The WO 01/28416 A1 discloses further a PDA comprising a battery, a display, connection means to physically connect the PDA with the at least one separate fixating device of the computer, identification means to recognise the at least one separate fixating device after or during fixation of the PDA at the at least one separate fixating device, a storage device for storing different graphical user interfaces and transmittal means to automatically transmit the graphical user interface belonging to the recognised fixating device to the display.

The disadvantage of the known multifunctional portable electronic devices is that the presented graphical user interface has to be adapted by the user himself, if the user wants to see something special on the display. The other disadvantage is that too much information is shown at the same time on the display.

The problem underlying the present invention is thus to provide a multifunctional portable electronic device and a method for transmitting a particular graphical user interface to the display of a multifunctional portable electronic device which are able to automatically adjust the graphical user interface on the display to match the specific activity of the user or the specific need of the user.

The invention is based on the finding that this problem can be solved by providing specific multifunctional portable electronic devices. The multifunctional portable electronic device can be built in that way that the display builds the main part of the device.

According to a first aspect the present invention relates to a multifunctional portable electronic device with the features of claim 1.

The core of the invention is that the graphical user interface adjusts automatically to the fixating device after or during the fixation of the multifunctional portable electronic device at the fixating device. The identification means recognises the separate fixating device. The transmittal means of the multifunctional portable electronic device comprises comparator means to compare the recognised separate fixating device with known fixating devices and transmits a specific graphical user interface belonging to the recognised fixating device to the display. Therefore the user of the multifunctional portable electronic device gets a specific graphical user interface in dependency of the recognised separate fixating device. The multifunctional portable electronic device automatically adjusts a specific graphical user interface to the needs of the user of the multifunctional portable electronic device.

The multifunctional portable electronic device comprises at least one separate sensor for measuring physical parameters. Furthermore the multifunctional portable electronic device comprises transmission means for receiving the measured physical parameters. The transmission means are advantageously wireless transmission means for a wireless transmission of the measured data from the at least one sensor to the multifunctional portable electronic device. The electrical power is been provided by a battery. After the automatically adjustment of the graphical user interface to the display of the multifunctional portable electronic device the user gets information belonging to the recognised separate fixating device. Advantageously the multifunctional portable electronic device comprises two or more different separate fixating devices. Depending on the separate fixating device, to which the multifunctional portable electronic device is been physically connected, to user gets a specific graphical user interface for the recognised separate fixating device on the display of the multifunctional portable electronic device. Depending on the presented graphical user interface these parameters are presented which belong to the graphical user interface.

The advantage of the multifunctional portable electronic device is the automatically adjustment of the graphical user interface on the display of the multifunctional portable electronic device in dependency of the separate fixating device. The multifunctional portable electronic device presents the desired needs to the user, without the need for doing an adjustment by the user himself. That means the user does not have to search a menu of the multifunctional portable electronic device for a certain graphical user interface. The multifunctional portable electronic device can give the user a choice between two or more graphical user interfaces belonging to he one recognised separate fixating device.

The connection means to physically connect the multifunctional portable electronic device with the at least one separate fixating device can be different. The connection can be tied positively, for example with a clamping connection, screw connection or the like. The connection can be form closed, as well.

The identification of the separate fixating device can be done in several different ways. According to one embodiment, the multifunctional portable electronic device is characterized in that the at least one separate fixating device comprises identification markings, wherein each fixating device has a individual identification marking, and the identification means comprises sensor means to recognise the identification markings of the at least one separate fixating device. The sensor means can be for example a pulse-, a proximity-, a pressure-, a position-, a photo-, a infrared or a measuring sensor. Furthermore the sensor means can be a circuit. The identification marking can be for example a barcode, a specific construction or a shortcut. Shortcuts can be read by certain circuits.

Preferred is a multifunctional portable electronic device, wherein the individual identification marking are arranged at connector pins at the at least one separate fixating device. The individual identification marking can be shortcuts on the connector pins, which can be read for example with a digital circuit. Each separate fixating device has different shortcuts on the connection pins representing the identification marking of that fixating device.

Another preferred multifunctional portable electronic device is characterized in that the at least one separate fixating device comprises a RFID-tag, which stores an individual identification marking, and the identification means of the multifunctional portable electronic device comprises an antenna and a transceiver for reading out the individual identification marking of the RFID-tag. RFID means Radio Frequency Identification. The antenna of the multifunctional portable electronic device emits radio signals to activate the RFID-tag of the separate fixating device and to read data from the tag. The transceiver of the multifunctional portable electronic device emits radio waves in ranges of anywhere from one inch to 100 feet or more, depending upon its power output and the radio frequency used. When the RFID-tag of a separate fixating device passes through the electromagnetic zone of the antenna und and the transceiver, respectively, it detects the transceiver's activation signal. The transceiver can decode the data encoded in the tag's integrated circuit and can pass the data to the multifunctional portable electronic device for processing.

RFID-tags come in a wide variety of shapes and sizes. RFID-tags are categorized as either active or passive. Active RFID-tags are powered by an internal battery. The battery-supplied power of an active tag generally gives it a longer read range. That means each separate fixating device has a RFID-Tag with it own battery.

Passive RFID-tags operate without a separate external power source and obtain operating power generated from the transceiver. Passive tags are consequently much lighter than active tags, less expensive, and offer a virtually unlimited operational lifetime. The trade off is that they have shorter read ranges than active tags and require a higher-powered reader. Read-only tags are typically passive and are programmed with a unique set of data that cannot be modified.

Therefore the RFID-tag of a separate fixating device is preferred a passive tag like a read-only tag.

The advantage of using a RFID-tag is the noncontact technology. The tags can be read through a variety of substances such as snow, fog, ice, paint, crusted grime, and other visually and environmentally challenging conditions, where barcodes or other optically read technologies would be useless. The identification marking can be read out during or after the physically connection of the multifunctional portable electronic device with a separate fixating device. Therefore, the user can get the graphical user interface as soon as possible.

The connection means of the multifunctional portable electronic device according to one embodiment comprises advantageously flexible spring, clip or clamp means.

The multifunctional portable electronic device is characterized in that the at least one separate fixating device is a separate holder, like a wristband or a holder for a handlebar or a belt or a chest belt. That means the multifunctional portable electronic device can be fixed at a wristband, at a holder for a handlebar, at belt, in particular a chest belt, or the like. Depending on the activity and the needs of the user the multifunctional portable electronic device can be fixed at one of the different separate fixating devices. If the fixating device is a holder for a handlebar of a bike, the identification means of the multifunctional portable electronic device recognises the holder for a handlebar of the bike and the transmittal means transmits the appropriate graphical bike user interface to the display of the multifunctional portable electronic device. The display can show the measured parameters belonging to the "graphical bike user interface". The parameters measured by the different sensors are received by the transmission means of the multifunctional portable electronic device. The sensors transmit the measured parameters and data preferably wireless to the transmission means of the multifunctional portable electronic device. The transmittal means picks these parameters out of a mass of parameters measured by several different sensors which belong to the graphical user interface presented on the display. The at least one graphical user interface matches to the at least one separate fixating device. It is possible that two or more graphical user interfaces match to one separate fixating device. In that case the user can choose between the graphical user interfaces. The graphical user interface can provide for example only the heard rate measured by a pulse sensor. On the other hand a graphical user interface can provide the heart rate of a person measured by a pulse sensor and the walking speed of the person measured by a speed sensor. The presentation of the data and measured parameters depends on the different graphical user interfaces stored in the storage device of the multifunctional portable electronic device.

The multifunctional portable electronic device comprises preferably a keypad. That allows the user to interact with the multifunctional portable electronic device. The user can change or can choose a graphical user interface by pressing a key. Based on a first graphical user interface belonging to one certain fixating device the user can vary the graphical user interface to his specific needs and wishes. For example, if the user rides a bike and gets a specific bike graphical user interface, the user can push a key to get only the speed parameters instead if the speed and the distance parameters.

The multifunctional portable electronic device has the advantage that the display presents automatically the graphical user interface which belongs to the separate fixating device where the multifunctional portable electronic device is fixed. If the multifunctional portable electronic device is connected to a wristband of a watch the graphical user interface provides for example the time and the date. If the multifunctional portable electronic device is connected to a wristband of a pulse monitor the graphical user interface provides the heartbeat, the pulse, the blood pressure or the like. If connected to a mobile telephone the graphical user interface provides telephone details. Being connected to a handle bar of a bike, the display presents automatically a graphical user interface of a bike tachometer. The multifunctional portable electronic device comprises a main unit comprising the at least one separate sensor, the transmission means, the battery, the at least one separate fixating device, and a detachable unit comprising a second battery, the display, connection means to physically connect the detachable unit with the at least one separate fixating device, identification means to recognise the at least one separate fixating device after fixation of the detachable unit at the at least one separate fixating device, the storage device for storing different graphical user interfaces and the transmittal means to automatically transmit the graphical user interface belonging to the recognised fixating device to the display.

The main unit, the detachable unit and the sensors communicate with each other through a wireless transmission of data. The detachable unit comprises these parts, which are necessary for presenting the graphical user interface. That means the detachable unit is small and light and therefore can easily being connected to a separate fixating device, like a wristband, a watch like holder, a handle bar holder, or the like. The detachable unit comprises mainly the display for providing the graphical user interface. The display builds the biggest part of the detachable unit. The detachable unit comprises a small battery for a stand-alone operating. The detachable unit comprises the connection means to physically connect the detachable unit with the at least one separate fixating device and to the main unit. The main unit is like a separate fixating device for the detachable unit. Further, the detachable unit comprises the identification means to recognise the at least one separate fixating device after or during fixation of the detachable unit at the at least one separate fixating device. The storage device for storing different graphical user interfaces and the transmittal means to automatically transmit the graphical user interface belonging to the recognised fixating device to the display are part of the detachable unit, as well.

A multifunctional portable electronic device like this enables a very large use of the multifunctional portable electronic device. Normally a multifunctional portable electronic device providing a lot of different features is a relatively large device and therefore can not be used very easily. The advantage of this multifunctional portable electronic device is that the main unit can be worn in a pocket near the body, while the detachable unit is fixed at a wristband or a holder of a handle bar, or the like. The detachable unit makes the multifunctional portable electronic device much more flexible than an electronic device comprising all features in one solution. During the connection or after being physically connected to the separate fixating device the display of the detachable unit automatically adjusts the graphical user interface to the separate fixating device to match the specific activity or wishes of the user.

The multifunctional portable electronic device integrates several information and communication needs, like playing music, calling, displaying a speed, a distance, a heart rate, etc., in a single device with a detachable unit that offers the relevant features in the right context at the right position. The detachable unit contains the display, a storage device for providing certain graphical user interfaces and can be attached to different holders. For running the separate fixating device is a watch like holder on the wrist, for cycling a handle bar holder. When the detachable unit is secured in the separate fixating device, the detachable unit automatically adjusts the graphical user interface to match the specific activity. The advantage is the reduction of the weight to be worn at the separate fixating device and therefore at the wrist or at the bike, for example. The multifunctional portable electronic device allows much more mobility. Furthermore, the cost for acquisition of several different devices can be saved. The only costs are the costs for the multifunctional portable electronic device, the several sensors and the different separate fixating devices. But this is still cheaper than the price for several different devices, because only one display, one transmission means and one storage means are needed.

Furthermore a multifunctional portable electronic device like this can integrate all functions, for example the music can be switched of if the telephone rings. There are no complicated modes on an all purpose device. When the detachable unit is attached to a specific separate fixating device, on a wristband for running or on a handle bar for cycling, the detachable unit switches automatically to the right mode for that separate fixating device. Back in the main unit, the detachable unit switches automatically back to the mode of the main unit. The main unit is preferably a mobile telephone or a PDA (PDA = Personal Digital Assistant). The detachable unit further comprises means for being detachable fixated at the main unit. The means for connecting the detachable unit to the main unit are advantageously the same connection means to physically connect the multifunctional portable electronic device with the at least one separate fixating device. The main unit is like a separate fixating unit to the detachable unit, with the difference that the main unit comprises more elements and features than a separate fixating device. This can be the main battery, the at least one separate sensor for measuring physical parameters and the transmission means for receiving the measured physical parameters.

Preferred is further a multifunctional portable electronic device, whereby the main unit is a mobile telephone or a PDA. The mobile telephone or the PDA can be used as a multifunctional portable electronic device, when being divided into main unit and a detachable unit. The main unit provides all features of a normal mobile telephone or PDA. But by detaching the detachable unit comprising the display the device can be used for a lot of other things, like a pulse monitor, a watch or a tachometer. The storage device for storing different graphical user interfaces is part of the main unit or of the detachable unit. That reduces the weight and the measures of the detachable unit. During or after the fixation of the detachable unit at a separate fixating device and after recognition of the separate fixating device through the identification means of the detachable unit, the detachable unit sends a request to the main unit asking for the graphical user interface belonging to the recognised separate fixating device. The main unit looks for the graphical user interface in the storage device and sends the relevant graphical user interface to the detachable unit. After receiving the relevant graphical user interface the transmittal means of the detachable unit transmits the received graphical user interface to the display of the detachable unit. The detachable unit has to fulfil following jobs: the connection with the different separate fixating devices; the identification of the separate fixating devices; providing the graphical user interface belonging to the recognised separate fixating device to the display of the detachable unit. The detachable unit can comprise the storage device for storing the different graphical user interfaces belonging to the different separate fixating devices. The storage device can also be part of the main unit.

In another preferred embodiment of the multifunctional portable electronic device, the multifunctional portable electronic device comprises an acoustic output interface. According to another aspect, the present invention relates to a method for transmitting a particular graphical user interface to the display of a multifunctional portable electronic device. The method comprises the features of claim 13.

The multifunctional portable electronic device, in particular the detachable unit of the multifunctional portable electronic device, is being fixed at one of the different separate fixating devices. During or after the connection the identification means of the multifunctional portable electronic device, in particular of the detachable unit of the multifunctional portable electronic device, identifies the separate fixating device. After being identified the transmittal means of the multifunctional portable electronic device, in particular of the detachable unit of the multifunctional portable electronic device, transmits the relevant graphical user interface belonging to the recognised separate fixating device to the display of the multifunctional portable electronic device, in particular of the detachable unit of the multifunctional portable electronic device. This method enables the automatically adjustment of the graphical user interface to the display depending on the separate fixating device, for example a handle bar holder, a wristband or a belt. The user has to do nothing by himself except for the fixation of the multifunctional portable electronic device, in particular of the detachable unit of the multifunctional portable electronic device, to one of the different fixating devices.

During or after the connection of the multifunctional portable electronic device, in particular of the detachable unit of the multifunctional portable electronic device, to the separate fixating device, the identification means looks for graphical user interfaces stored in the storage means of the multifunctional portable electronic device, in particular of the detachable unit of the multifunctional portable electronic device, belonging to the recognised separate fixating device. If a graphical user interface matches to the recognised separate fixating device the transmittal means transmits this graphical user interface to the display. The user gets therefore exactly that what he wants to see. He does not have to do something by himself.

The method is characterised in that, that after the recognition of the separate fixating device the detachable unit sends a request to the main unit, asking for the graphical user interface belonging to the recognised separate fixating device, and after receiving the request, the main unit sends the relevant graphical user interface to the detachable unit, and after receiving the relevant graphical user interface the transmittal means automatically transmits the graphical user interface belonging to the recognised separate fixating device to the display.

In this case the storage means, where the different graphical user interfaces are being stored, are part of the main unit of the multifunctional portable electronic device. The detachable unit has to ask for the relevant graphical user interface at the main unit after recognising the separate fixating device. The transmission of the request and the graphical user interface can be done by the transmission means, which transmits the sensor measured parameters to the detachable unit.

Features and advantages described in context with the multifunctional portable electronic device also apply to the inventive method and vice versa.

The invention will now be described again in detail with reference to the enclosed drawings, wherein:
- Figure 1:: shows a schematic depiction of a multifunctional portable electronic device comprising a main unit, a detachable unit and several sensors;
- Figure 2:: shows a schematic depiction of a detachable unit and a separate fixating device;
- Figure 3:: shows a schematic depiction of an athlete wearing a multifunctional portable electronic device according to the present invention.

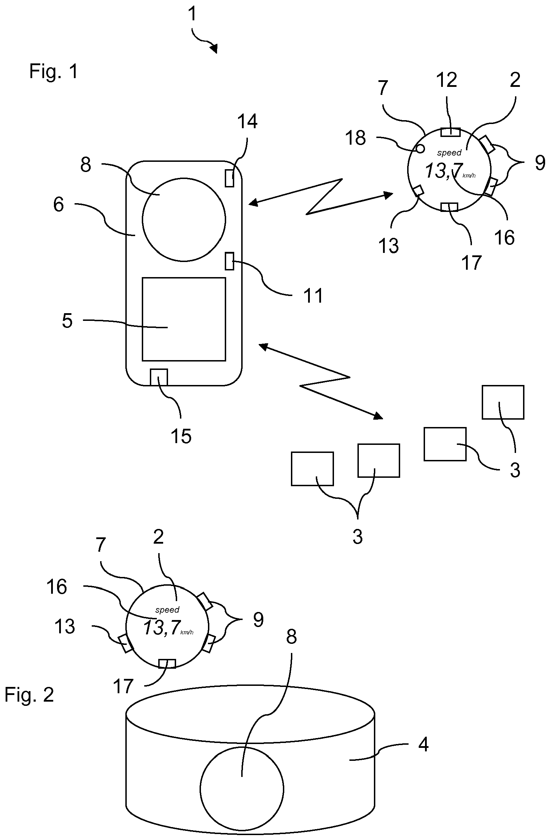

Fig. 1 shows a schematic depiction of a multifunctional portable electronic device 1 comprising a main unit 6, a detachable unit 7 and several separate sensors 3. The main unit 6 comprises a keypad 5, connections means 8 for fixating the detachable unit 7 at the main unit 6, transmission means 14 for receiving the measured physical parameters from the sensors 3, a battery 15 and a storage device 11 for storing the different graphical user interfaces 16 belonging to the several separate fixating devices 14.

The detachable unit 7 comprises a display 2 for presenting the graphical user interface 16, a second small battery 17 for stand-alone-operation of the detachable unit 7, connection means 8, which are not shown, for the fixation of the detachable unit 7 at a separate fixating device 4, keys or a keypad 9, identification means 12 for recognising the at least one separate fixating device 4 after or during the fixation of the detachable unit 7 at the at least one separate fixating device 4, and transmittal means 13 to automatically transmit the graphical user interface 16 belonging to the recognised fixating device 4 to the display 2. Further, the detachable unit 7 comprises an acoustic output interface 18, for example a loudspeaker.

The advantage of the multifunctional portable electronic device 1 is the automatically adjustment of the graphical user interface 16 on the display 2 of detachable unit 7 in dependency of the recognised separate fixating device 4. The display 2 of the detachable unit 7 presents the desired needs to the user, without the need for doing an adjustment by the user himself. That means the user does not have to search a menu of the multifunctional portable electronic device 1 for a certain graphical user interface 16. The date and parameters measured by the sensors 3 are automatically presented at the graphical user interface 16. That means only these data and parameters are presented which can be shown with the elected certain graphical user interface 16.

The multifunctional portable electronic device 1 has the advantage that the display 2 of the detachable unit 7 presents automatically the graphical user interface 16 which belongs to the separate fixating device 4 where the detachable unit 7 is fixed. If the detachable unit 7 is connected to a wristband of a watch the graphical user interface 16 provides for example the time and the date. If the detachable unit 7 is connected to a wristband of a pulse monitor the graphical user interface 16 provides the heartbeat, the pulse, the blood pressure or the like. If connected to a mobile telephone the graphical user interface 16 provides telephone details. Being connected to a handle bar of a bike, the display 2 presents automatically a graphical user interface 16 of a bike tachometer.

The main unit 6, the detachable unit 7 and the sensors 3 communicate with each other through a wireless transmission of data. The detachable unit 7 is small and light and therefore can easily being connected to a separate fixating device 4, like a wristband, a watch like holder, a handle bar holder, or the like. The detachable unit 7 comprises mainly the display 2 for providing the graphical user interface 16. That means the display 2 builds the biggest part of the detachable unit 7. The detachable unit comprises a small battery 17 for a stand-alone operating. The detachable unit 7 comprises connection means 8 to physically connect the detachable unit 7 to the at least one separate fixating device 4 or to the main unit 6. The identification means 12 of the detachable unit 7 recognises a separate fixating device 4 after or during the fixation of the detachable unit 7 at the separate fixating device 4.

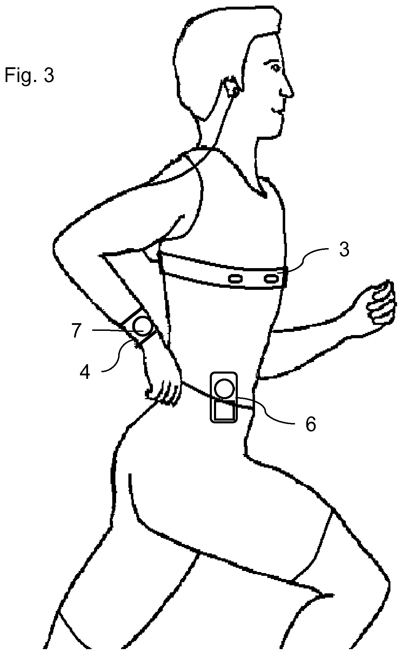

The advantage of the multifunctional portable electronic device 1 is that the main unit 6 can be worn in a pocket near the body, while the detachable unit 7 is fixed for example at a wristband, as shown in fig. 3. The sensor 3 providing the data and parameters is a sensor placed in a chest belt in this embodiment. The detachable unit 7 makes the multifunctional portable electronic device 1 much more flexible than an electronic device comprising all features in one solution. During the connection or after being physically connected to the separate fixating device 4 the display 2 of the detachable unit 7 automatically adjusts the graphical user interface 16 belonging to the separate fixating device 4 to match the specific activity or wishes of the user.

The detachable unit 7 can be attached to different holders. As shown in fig. 2 the detachable unit 7 can be connected to a wristband 4. The wristband 4 comprises connection means 8 for the physically connection of the detachable unit 7 at the wristband 4. For running the separate fixating device 4 can be a watch like holder on the wrist and for cycling a handle bar holder.

When the detachable unit 7 is attached to a specific separate fixating device 4 the detachable unit 7 switches automatically to the right mode for that separate fixating device 4. Back in the main unit 6, the detachable unit 7 switches automatically back to the mode of the main unit 6. The main unit 6 is preferably a mobile telephone or a PDA.

The storage device 11 for storing different graphical user interfaces 16 is in the embodiment shown in fig. 1 part of the main unit 6. That reduces the weight and the measures of the detachable unit 7. During or after the fixation of the detachable unit 7 at a separate fixating device 4 and after recognition of the separate fixating device 4 through the identification means 12 of the detachable unit 7, the detachable unit 7 sends a request to the main unit 6 asking for the special graphical user interface 16 belonging to the recognised separate fixating device 4. The main unit 6 looks for the graphical user interface 16 in the storage device 11 and sends the relevant graphical user interface 16 to the detachable unit 7. After receiving the relevant graphical user interface 16 the transmittal means 13 of the detachable unit 7 transmits the received graphical user interface 16 to the display 2 of the detachable unit 7.

The detachable unit 7 can also comprise the storage device 11 for storing the different graphical user interfaces 16 belonging to the different separate fixating devices 4.

### Reference Numbers

- 1: multiple portable electronic device
- 2: display
- 3: sensors
- 4: separate fixating device
- 5: keypad
- 6: main unit
- 7: detachable unit
- 8: connection means
- 9: keypad of detachable unit

- 11: storage device
- 12: identification means
- 13: transmittal means
- 14: transmission means
- 15: battery
- 16: graphical user interface
- 17: battery of detachable unit
- 18: acoustic output interface

## Claims

1. Multifunctional portable electronic device comprising
a main unit (6), comprising at least one separate sensor (3) for measuring physical parameters, transmission means (14) for receiving the measured physical parameters, a battery (15), and
at least one separate fixating device (4), and
a detachable unit (7) comprising a second battery (17), a display (2), connection means (8) to physically connect the detachable unit (7) with the at least one separate fixating device (4),
identification means (12) to recognise the at least one separate fixating device (4) after or during fixation of the detachable unit (7) at the at least one separate fixating device (4),
a storage device (11) for storing different graphical user interfaces (16) and transmittal means (13) to automatically transmit the graphical user interface (16) belonging to the recognised fixating device (4) to the display (2), wherein the main unit (6) and the detachable unit (7) are adapted to communicate with each other through a wireless transmission of data,
that-the main unit (6) is adapted to transmit the sensor measured parameters to the detachable unit (7)
the fixation device (4) is a separate holder for the detachable unit (7) and comprises identification markings, wherein each separate fixating device (4) has a individual identification marking,
the main unit (6) comprises connection means (8) for fixating the detachable unit (7) at the main unit (6), and the storage device (11) for storing different graphical user interfaces (16) is part of the main unit (6).

2. Multifunctional portable electronic device according to claim 1, **characterized in that** the at least one separate fixating device (4) comprises identification markings, wherein each separate fixating device (4) has a individual identification marking, and the identification means (12) comprises sensor means to recognise the identification markings of the at least one separate fixating device (4).

3. Multifunctional portable electronic device according to claim 2, **characterized in that** the individual identification marking are arranged at connector pins at the at least one separate fixating device (4).

4. Multifunctional portable electronic device according to claim 1, **characterized in that** the at least one separate fixating device (4) comprises a RFID-tag, which stores an individual identification marking, and the identification means (12) of the multifunctional portable electronic device (1) comprises an antenna and a transceiver for reading out individual identification marking of the RFID-tag.

5. Multifunctional portable electronic device according to anyone of claims 1 to 4, **characterized in that** the connection means (8) comprises flexible spring, clip or clamp means.

6. Multifunctional portable electronic device according to anyone of claims 1 to 5, **characterized in that** the at least one separate fixating device (4) is a wristband or a holder for a handlebar or a belt or a chest belt.

7. Multifunctional portable electronic device according to anyone of claims 1 to 6, **characterized in that** the transmission means (14) are means for wireless transmission of data.

8. Multifunctional portable electronic device according to anyone of claims 1 to 7, **characterized in that** at least one graphical user interface (16) matches to the at least one separate fixating device (4).

9. Multifunctional portable electronic device according to anyone of claims 1 to 8, **characterized in that** the multifunctional portable electronic device (1) comprises a keypad (5).

10. Multifunctional portable electronic device according to anyone of the claims 1 to 9, **characterized in that** the detachable unit (7) comprises means (8) for being detachable fixated at the main unit (6).

11. Multifunctional portable electronic device according to anyone of the claims 1 to 10, **characterized in that** the main unit (6) is a mobile telephone or a PDA.

12. Multifunctional portable electronic device according to anyone of the claims 1 to 11, **characterized in that** the multifunctional portable electronic device (1) comprises an acoustic output interface (18).

13. Method for transmitting a particular graphical user interface to the display (2) of a multifunctional portable electronic device (1), wherein the multifunctional portable electronic device (1) is constructed according to one of the claims 1 to 12, wherein the detachable unit (7) is detached from connection means (8) of a main unit (6) of the multifunctional portable electronic device (1) being physically connected to the at least one separate fixation device (4), wherein after or during the connection identification means (12) recognises the at least one separate fixating device (4) and after the recognition the transmittal means (13) of the detachable unit (7) automatically transmits the graphical user interface (16) belonging to the recognised separate fixating device (4) to the display (2) of the detachable device (7) and sensor measured parameters are transmitted to the detachable unit (7) from the main unit (6), wherein after the recognition of the separate fixating device (4) the detachable unit (7) sends a request to the main unit (6), asking for the graphical user interface (16) belonging to the recognised separate fixating device (4), and after receiving the request, the main unit (6) sends the relevant graphical user interface (16) to the detachable unit (7), and after receiving the relevant graphical user interface (16) the transmittal means (13) automatically transmits the graphical user interface (16) belonging to the recognised separate fixating device (4) to the display (2).

## Patentansprüche

1. Multifunktionale tragbare elektronische Vorrichtung umfassend:
eine Haupteinheit (6), die zumindest einen separaten Sensor (3) zum Messen physikalischer Parameter, Übermittlungsmittel (14) zum Empfang der gemessenen physikalischen Parameter, eine Batterie (15), umfasst, und zumindest eine separate Befestigungsvorrichtung (4), und
eine abnehmbare Einheit (7) umfassend eine zweite Batterie (17), eine Anzeige (2), Verbindungsmittel (8) zum physikalischen Verbinden der abnehmbaren Einheit (7) mit zumindest einer separaten Befestigungsvorrichtung (4), Identifikationsmittel (12) zum Erkennen der zumindest einen separaten Befestigungsvorrichtung (4) nach oder während der Befestigung der abnehmbaren Einheit (7) an der zumindest einen separaten Befestigungsvorrichtung (4),
eine Speichervorrichtung (11) zum Speichern unterschiedlicher graphischer Benutzeroberflächen (16) und Übertragungsmittel (13) zum automatischen Übertragen der graphischen Benutzeroberfläche (16), die zu der erkannten Befestigungsvorrichtung (4) gehört, an die Anzeige (2),
wobei die Haupteinheit (6) und die abnehmbare Einheit (7) dazu ausgelegt sind miteinander durch drahtlose Übermittlung von Daten zu kommunizieren,
die Haupteinheit (6) dazu ausgelegt ist die vom Sensor gemessenen Parameter an die abnehmbare Einheit (7) zu übertragen,
die Befestigungsvorrichtung (4) ein separater Halter für die abnehmbare Einheit (7) ist und Identifikationsmarkierungen umfasst, wobei jede separate Befestigungsvorrichtung (4) eine individuelle Identifikationsmarkierung hat,
die Haupteinheit (6) Verbindungsmittel (8) zur Befestigung der abnehmbaren Einheit (7) an der Haupteinheit (6) aufweist, und
die Speichervorrichtung (11) zum Speichern unterschiedlicher graphischer Benutzeroberflächen (16) Teil der Haupteinheit (6) ist.

2. Multifunktionale tragbare elektronische Vorrichtung gemäß Anspruch 1, **dadurch gekennzeichnet, dass** die zumindest eine separate Befestigungsvorrichtung (4) Identifikationsmarkierungen umfasst, wobei jede separate Befestigungsvorrichtung (4) eine individuelle Identifikationsmarkierung hat und die Identifikationsmittel (12) Sensormittel umfassen, um die Identifikationsmarkierungen von der mindestens einen separaten Befestigungsvorrichtung (4) zu erkennen.

3. Multifunktionale tragbare elektronische Vorrichtung gemäß Anspruch 2, **dadurch gekennzeichnet, dass** die individuelle Identifikationsmarkierungen an Verbindungsstiften an der mindestens einen separaten Befestigungsvorrichtung (4) angeordnet sind.

4. Multifunktionale tragbare elektronische Vorrichtung gemäß Anspruch 1, **dadurch gekennzeichnet, dass** die zumindest eine separate Befestigungsvorrichtung (4) einen RFID-Tag umfasst, der eine individuelle Identifikationsmarkierung speichert, und die Identifikationsmittel (12) der multifunktionalen tragbaren elektronischen Vorrichtung (1) eine Antenne und einen Transciever zum Auslesen individueller Identifikationsmarkierungen des RFID-Tags umfasst.

5. Multifunktionale tragbare elektronische Vorrichtung gemäß einem der Ansprüche 1 bis 4, **dadurch gekennzeichnet, dass** die Verbindungsmittel (8) flexible Federn, Clip oder Klemmmittel umfassen.

6. Multifunktionale tragbare elektronische Vorrichtung gemäß einem der Ansprüche 1 bis 5, **dadurch gekennzeichnet, dass** die zumindest eine separate Befestigungsvorrichtung (4) ein Armband oder ein Halter für eine Lenkstange oder ein Gürtel oder ein Brustgürtel ist.

7. Multifunktionale tragbare elektronische Vorrichtung gemäß einem der Ansprüche 1 bis 6, **dadurch gekennzeichnet, dass** die Übermittlungsmittel (14) Mittel zur drahtlosen Übermittlung von Daten sind.

8. Multifunktionale tragbare elektronische Vorrichtung gemäß einem der Ansprüche 1 bis 7, **dadurch gekennzeichnet, dass** zumindest eine graphische Benutzeroberfläche (16) mit der zumindest einen separaten Befestigungsvorrichtung (4) zusammenpasst.

9. Multifunktionale tragbare elektronische Vorrichtung gemäß einem der Ansprüche 1 bis 8, **dadurch gekennzeichnet, dass** die multifunktionale tragbare elektronische Vorrichtung (1) ein Tastenfeld (5) umfasst.

10. Multifunktionale tragbare elektronische Vorrichtung gemäß einem der Ansprüche 1 bis 9, **dadurch gekennzeichnet, dass** die abnehmbare Einheit (7) Mittel (8) umfasst, um an der Haupteinheit (6) abnehmbar befestigt zu sein.

11. Multifunktionale tragbare elektronische Vorrichtung gemäß einem der Ansprüche 1 bis 10, **dadurch gekennzeichnet, dass** die Haupteinheit (6) ein Mobiltelefon oder ein PDA ist.

12. Multifunktionale tragbare elektronische Vorrichtung gemäß einem der Ansprüche 1 bis 11, **dadurch gekennzeichnet, dass** die multifunktionale tragbare elektronische Vorrichtung (1) eine akustische Ausgabeschnittstelle (18) umfasst.

13. Verfahren zum Übermitteln einer bestimmten graphischen Benutzeroberfläche an die Anzeige (2) einer multifunktionalen tragbaren elektronischen Vorrichtung (1), wobei die multifunktionale tragbare elektronische Vorrichtung (1) gemäß einem der Ansprüche 1 bis 12 aufgebaut ist, wobei die abnehmbare Einheit (7) von den Verbindungsmitteln (8) einer Haupteinheit (6) der multifunktionalen tragbaren elektronischen Vorrichtung (1), die physikalisch mit der zumindest einen separaten Befestigungsvorrichtung (4) verbunden ist, abgenommen wird, nach oder während der Verbindung Identifikationsmittel (12) die zumindest eine separate Befestigungsvorrichtung (4) erkennen und nach dem Erkennen die Übertragungsmittel (13) der abnehmbaren Einheit (7) automatisch die graphische Benutzeroberfläche (16), die zu der erkannten separaten Befestigungsvorrichtung (4) gehört, an die Anzeige (2) der abnehmbaren Einheit (7) übermittelt und wobei vom Sensor gemessene Parameter an die abnehmbare Einheit (7) der Haupteinheit (6) übertragen werden, wobei nach dem Erkennen der separaten Befestigungsvorrichtung (4) die abnehmbare Einheit (7) eine Anfrage an die Haupteinheit (6) sendet, in der nach der graphischen Benutzeroberfläche (16), die zu der erkannten separaten Befestigungsvorrichtung (4) gehört, gefragt wird, und nach dem Erhalt der Anfrage die Haupteinheit (6) die relevante graphische Benutzeroberfläche (16) an die abnehmbare Einheit (7) sendet und nach Erhalt der relevanten graphischen Benutzeroberfläche (16) die Übertragungsmittel (13) automatisch die graphische Benutzeroberfläche (16), die zu der erkannten separaten Befestigungsvorrichtung (4) gehört an die Anzeige (2) übermitteln.

## Revendications

1. Dispositif électronique multifonction portable comprenant :
une unité principale (6), comprenant au moins un capteur séparé (3) destiné à mesurer des paramètres physiques, un moyen de transmission (14) destiné à recevoir les paramètres physiques mesurés, une batterie (15), et
au moins un dispositif de fixation séparé (4), et
une unité amovible (7) comprenant une seconde batterie (17), un dispositif d'affichage (2), un moyen de liaison (8) destiné à relier physiquement l'unité amovible (7) avec le ou les dispositifs de fixation séparés (4),
un moyen d'identification (12) destiné à identifier le ou les dispositifs de fixation séparés (4) après ou pendant la fixation de l'unité amovible (7) sur le ou les dispositifs de fixation séparés (4),
un dispositif de mémorisation (11) destiné à mémoriser différentes interfaces graphiques d'utilisateur (16) et un moyen de transmission (13) destiné à transmettre automatiquement l'interface graphique d'utilisateur (16) appartenant au dispositif de fixation identifié (4) vers le dispositif d'affichage (2),
dans lequel l'unité principale (6) et l'unité amovible (7) sont adaptées pour communiquer l'une avec l'autre par l'intermédiaire d'une transmission de données sans fil, l'unité principale (6) est adaptée pour transmettre les paramètres mesurés par capteur à l'unité amovible (7),
le dispositif de fixation (4) est un élément de retenue séparé pour l'unité amovible (7) et comprend des repères d'identification, dans lequel chaque dispositif de fixation séparé (4) comporte un repère d'identification individuel,
l'unité principale (6) comprend un moyen de liaison (8) destiné à fixer l'unité amovible (7) sur l'unité principale (6), et
le dispositif de mémorisation (11) destiné à mémoriser différentes interfaces d'utilisateur graphiques (16) fait partie de l'unité principale (6).

2. Dispositif électronique multifonction portable selon la revendication 1, **caractérisé en ce que** le ou les dispositifs de fixation séparés (4) comprennent des repères d'identification, dans lequel chaque dispositif de fixation séparé (4) présente un repère d'identification individuel, et le moyen d'identification (12) comprend un moyen formant capteur pour identifier les repères d'identification du ou des dispositifs de fixation séparés (4).

3. Dispositif électronique multifonction portable selon la revendication 2, **caractérisé en ce que** les repères d'identification individuels sont agencés au niveau de broches de connecteur sur le ou les dispositifs de fixation séparés (4).

4. Dispositif électronique multifonction portable selon la revendication 1, **caractérisé en ce que** le ou les dispositifs de fixation séparés (4) comprennent une étiquette RFID, qui mémorise un repère d'identification individuel, et le moyen d'identification (12) du dispositif électronique multifonction portable (1) comprend une antenne et un transmetteur pour extraire le repère d'identification individuel de l'étiquette RFID.

5. Dispositif électronique multifonction portable selon l'une quelconque des revendications 1 à 4, **caractérisé en ce que** les moyens de liaison (8) comprennent des moyens formant ressort, bagues ou pinces flexibles.

6. Dispositif électronique multifonction portable selon l'une quelconque des revendications 1 à 5, **caractérisé en ce que** le ou les dispositifs de fixation séparés (4) sont constitués par un bracelet ou un élément de retenue pour un guidon ou une ceinture ou une sangle abdominale.

7. Dispositif électronique multifonction portable selon l'une quelconque des revendications 1 à 6, **caractérisé en ce que** les moyens de transmission (14) sont des moyens de transmission de données sans fil.

8. Dispositif électronique multifonction portable selon l'une quelconque des revendications 1 à 7, **caractérisé en ce qu'**au moins une interface graphique d'utilisateur (16) est adaptée au ou aux dispositifs de fixation séparés (4).

9. Dispositif électronique multifonction portable selon l'une quelconque des revendications 1 à 8, **caractérisé en ce que** le dispositif électronique multifonction portable (1) comprend un clavier (5).

10. Dispositif électronique multifonction portable selon l'une quelconque des revendications 1 à 9, **caractérisé en ce que** l'unité amovible (7) comprend un moyen (8) destiné à être fixé de manière amovible sur l'unité principale (6).

11. Dispositif électronique multifonction portable selon l'une quelconque des revendications 1 à 10, **caractérisé en ce que** l'unité principale (6) est un téléphone mobile ou un assistant numérique PDA.

12. Dispositif électronique multifonction portable selon l'une quelconque des revendications 1 à 11, **caractérisé en ce que** le dispositif électronique multifonction portable (1) comprend une interface de sortie acoustique (18).

13. Procédé de transmission d'une interface graphique d'utilisateur particulière vers le dispositif d'affichage (2) d'un dispositif électronique multifonction portable (1), dans lequel le dispositif électronique multifonction portable (1) est construit selon l'une des revendications 1 à 12,
dans lequel l'unité amovible (7) est séparée du moyen de liaison (8) d'une unité principale (6) du dispositif électronique multifonction portable (1) qui est relié physiquement au ou aux dispositifs) de fixation séparé(s) (4), dans lequel, après ou pendant que le moyen d'identification de liaison (12) identifie le ou les dispositifs de fixation séparés (4) et, après l'identification, le moyen de transmission (13) de l'unité amovible (7) transmet automatiquement l'interface graphique d'utilisateur (16) appartenant au dispositif de fixation séparé (4) identifié vers le dispositif d'affichage (2) du dispositif amovible (7), et dans lequel les paramètres mesurés par capteur sont transmis à l'unité amovible (7) à partir de l'unité principale (6), dans lequel après l'identification du dispositif de fixation séparé (4), l'unité amovible (7) envoie une requête à l'unité principale (6) demandant l'interface graphique d'utilisateur (16) appartenant au dispositif de fixation séparé (4) identifié et, après réception de la requête, l'unité principale (6) envoie l'interface graphique d'utilisateur (16) concernée à l'unité amovible (7) et, après réception de l'interface graphique d'utilisateur (16) concernée, le moyen de transmission (13) transmet automatiquement l'interface graphique d'utilisateur (16) appartenant au dispositif de fixation séparé (4) identifié vers le dispositif d'affichage (2).
